# EUROPÄISCHE PATENTSCHRIFT

(11) **EP 1 339 444 B1**
(45) Veröffentlichungstag und Bekanntmachung des Hinweises auf die Patenterteilung: **29.09.2004**
(21) Anmeldenummer: 01999399.7
(22) Anmeldetag: 06.12.2001
(51) Int. Cl.: A61M 5/14

(54) **INFUSIONSHALTER ZUR ANBRINGUNG AN EINEN PATIENTEN TRAGENDEN EINRICHTUNGEN**
INFUSION HOLDER FOR MOUNTING ON DEVICES THAT SUPPORT A PATIENT
SUPPORT DE PERFUSION A MONTER SUR DES DISPOSITIFS SOUTENANT UN PATIENT

(30) Priorität: 09.12.2000 DE 20020925 U
(43) Veröffentlichungstag der Anmeldung: 03.09.2003
(73) Patentinhaber: Schulze, Michael, 48291 Telgte (DE)
(72) Erfinder: Schulze, Michael, 48291 Telgte (DE)
(74) Vertreter: Linnemann, Winfried
(86) Internationale Anmeldenummer: PCT/EP2001/014322
(87) Internationale Veröffentlichungsnummer: WO 2002/045782

(56) Entgegenhaltungen:
- EP-A- 0 167 345
- WO-A-96/03917
- DE-U- 1 950 302
- GB-A- 2 307 727
- US-A- 4 843 530
- US-A- 5 501 682
- US-A- 5 772 162
- US-A- 5 820 086
- US-A- 5 829 723

## Beschreibung

Die vorliegende Erfindung betrifft einen Infusionshalter zur Anbringung an einen Patienten tragenden Einrichtungen, wie Krankentragen oder -betten, mit einer aus einem oberen und unteren Säulenteil bestehenden Tragsäule, die an ihrem unteren Ende ein Kupplungselement zur Verbindung mit der den Patienten tragenden Einrichtung und an ihrem oberen Ende mindestens ein Halteelement für eine Infusionsflasche aufweist.

Ein Infusionshalter der vorstehend genannten Art ist aus DE-U 1 950 302 bekannt. Als Halteelement ist an diesem bekannten Infusionshalter bevorzugt eine Anordnung von zwei U-förmig gebogenen Haken vorgesehen, an denen Infusionsflaschen aufgehängt und/oder Infusionsschläuche geführt werden können.

Als nachteilig wird bei diesem bekannten Infusionshalter angesehen, daß in vielen praktischen Fällen die gebotenen Möglichkeiten für das Aufhängen von Infusionsflaschen und/oder für das Führen von Infusionsschläuchen nicht ausreichend sind. Dies führt dazu, daß beispielsweise bei einem Krankentransport mittels einer Trage oder eines Krankenbetts Teile der medizinischen Behandlungseinrichtungen, die mit dem auf der Trage transportierten Patienten mitgeführt werden müssen, vorübergehend lose auf der Trage oder dem Bett oder sogar auf dem Patienten selbst liegen. Dabei besteht die große Gefahr, daß diese Teile von der Trage herunterfallen, was zur Unterbrechung einer lebenswichtigen Infusion führen kann. Dies kann nur vermieden werden, wenn das den Krankentransport begleitende medizinische Personal auch noch einen Teil ihrer Aufmerksamkeit auf die auf der Trage oder dem Bett oder dem Patienten liegenden Teile der medizinischen Behandlungseinrichtungen richtet, was aber von Nachteil für den Patienten ist.

Für die vorliegende Erfindung stellt sich deshalb die Aufgabe, einen Infusionshalter der eingangs genannten Art zu schaffen, der die dargelegten Nachteile vermeidet und der insbesondere verbesserte und erweiterte Möglichkeiten zur Halterung von Teilen von medizinischen Behandlungseinrichtungen bietet.

Die Lösung dieser Aufgabe gelingt erfindungsgemäß mit einem Infusionshalter der oben genannten Art, der dadurch gekennzeichnet ist,
- daß er zusätzlich mit einem vom oberen Ende der Tragsäule ausgehenden, in seinem Verlauf verstellbaren, gelenkigen oder biegsamen schwanenhals ausgestattet ist,
- daß der Schwanenhals an seinem freien Ende ein oder mehrere weitere Halteelemente insbesondere für einen Beatmungsschlauch und/oder für eine Infusionsflasche und/oder für andere Teile einer medizinischen Behandlungseinrichtung, aufweist,
- daß die Tragsäule rohrförmig mit einem hohlen Inneren ausgeführt und oben offen ist und
- daß der Schwanenhals in gestreckter Stellung in das Innere der Tragsäule einschiebbar und aus dieser herausziehbar ist.

Mit dem erfindungsgemäßen Infusionshalter wird vorteilhaft die Möglichkeit geschaffen, nicht nur ein oder zwei Infusionsflaschen an dem Infusionshalter haltern und z.B. zusammen mit dem Patienten und der diesen tragenden Trage zu bewegen, sondern zusätzlich weitere Teile von medizinischen Behandlungseinrichtungen zu haltern und/oder zu führen. Dazu kommt der Vorteil, daß der Schwanenhals in seinem Verlauf verstellbar und so an jeweilige Erfordernisse hinsichtlich der Positionierung und des Verlaufs von Teilen der medizinischen Behandlungseinrichtungen schnell und einfach angepaßt werden kann. Durch das Kupplungselement kann der Infusionshalter schnell und einfach mit einer einen Patienten tragenden Einrichtung verbunden werden. Wenn der Infusionshalter nicht mehr benötigt wird, kann er ebenso einfach wieder von der den Patienten tragenden Einrichtung abgebaut werden, indem das Kupplungselement gelöst wird. Die Ausgestaltung und räumliche Anordnung des vom oberen Ende der Tragsäule ausgehenden Schwanenhalses bietet den Vorteil, daß der Schwanenhals eine besonders große räumliche Reichweite erhält und daß er gleichzeitig nicht mit am oberen Ende der Tragsäule gehalterten Infusionsflaschen und von diesen ausgehenden Infusionsschläuchen kollidieren kann. Die Tatsache, daß die Tragsäule rohrförmig mit einem hohlen Inneren ausgeführt und oben offen ist und daß der Schwanenhals in gestreckter Stellung in das Innere der Tragsäule einschiebbar und aus dieser herausziehbar ist, bietet die vorteilhafte Möglichkeit, den Schwanenhals fast vollständig in das Innere der Tragsäule einzuschieben, wenn er nicht benötigt wird. Es verbleibt dann außerhalb der Tragsäule nur noch das obere Ende des Schwanenhalses mit den dort angeordneten Halteelementen. Diese können auch in der eingeschobenen Stellung des Schwanenhalses noch genutzt werden.

Ein weiterer Beitrag zur flexiblen Anpassung des Infusionshalters an die jeweils vorliegenden Erfordernisse besteht darin, daß vorzugsweise der Schwanenhals in unterschiedlichen Ausziehstellungen relativ zur Tragsäule, durch ein Feststellmittel fixierbar ist. Damit kann der Schwanenhals auch in einer nur teilweise aus der Tragsäule herausgezogenen Stellung sicher fixiert werden, so daß sich keine unerwünschten selbsttätigen Verstellungen des Schwanenhalses und der an dessen Halteelementen gehalterten Teile von medizinischen Behandlungseinrichtungen ergeben können. Als Feststellmittel für den Schwanenhals genügt im einfachsten Fall eine Klemmschraube, die im oberen Ende der Tragsäule in einer Gewindebohrung geführt ist.

Weiter ist bevorzugt vorgesehen, daß die Tragsäule als Teleskoprohr mit mindestens zwei ineinander geführten und gegeneinander verschiebbaren sowie fixierbaren Rohrabschnitten ausgebildet ist. Auf diese Weise ist die Länge der Tragsäule veränderbar, was ebenfalls zu einer guten Anpassung des Infusionshalters an die jeweiligen Bedürfnisse bei seinem Einsatz beiträgt. Außerdem läßt sich so der Infusionshalter auf ein relativ kleines Maß zusammenschieben, wenn er nicht benötigt wird. In diesem zusammengeschobenen Zustand kann er dann platzsparend untergebracht und gelagert werden.

Weiterhin ist bevorzugt das Kupplungselement mit einem äußeren, den unteren Tragsäulenteil bildenden Rohrabschnitt verbunden und das an der Tragsäule vorgesehene Halteelement an einem inneren, den oberen Tragsäulenteil bildenden Rohrabschnitt angebracht. Auf diese Weise wird erreicht, daß der bewegliche, obere Teil der Tragsäule im Inneren des unteren, feststehenden Teils der Tragsäule geführt ist, so daß bei der Verstellung der Länge der teleskopierbaren Tragsäule praktisch keinerlei Verletzungsgefahren für das Personal oder für einen Patienten bestehen. Außerdem ist so die Tragsäule in ihrem am stärksten belasteten unteren Teil aufgrund des dort größeren Durchmessers bedarfsgerecht stabiler.

Weiterhin ist vorgesehen, daß die Tragsäule und das Kupplungselement mittels eines feststellbaren Schwenkgelenks miteinander verbunden sind, wobei die Schwenkachse des Gelenks senkrecht zur Längsrichtung der Tragsäule verläuft und wobei in dem an einer einen Patienten tragenden Einrichtung angebrachten Zustand des Infusionshalters die Tragsäule um die Gelenk-Schwenkachse um mindestens 90° zwischen einer etwa vertikal nach oben verlaufenden Benutzungsstellung und einer etwa horizontalen, parallel zu der den Patienten tragenden Einrichtung verlaufenden Nichtbenutzungsstellung verschwenkbar ist. Diese Ausgestaltung des Infusionshalters bietet die vorteilhafte Möglichkeit, die Tragsäule bei Nichtbenutzung in eine waagerechte Lage zu verschwenken, in der der komplette Infusionshalter platzsparend parallel unmittelbar neben der den Patienten tragenden Einrichtung, wie Krankentrage oder -bett, verläuft. Da der Infusionshalter in dieser Stellung in keiner Weise störend in Erscheinung tritt, kann er auch an der den Patienten tragenden Einrichtung verbleiben, wenn er nicht benötigt wird. Dadurch ist er gleichzeitig bei Bedarf sehr schnell wieder in seine Benutzungsstellung bringbar, indem die Tragsäule wieder relativ zum Kupplungsstück in eine vertikale Stellung verschwenkt wird und danach durch Feststellung des Gelenks arretiert wird.

In diesem Zusammenhang ist bevorzugt vorgesehen, daß zumindest in der Benutzungsstellung der Tragsäule bei festgestelltem Gelenk zwei Teile des Gelenks in einem formschlüssigen Eingriff miteinander stehen. Auf diese Weise ist gewährleistet, daß die Tragsäule auch bei möglicherweise in der Hektik eines Einsatzes nicht ganz festgezogenem Feststellmittel des Gelenks gegen ein unerwünschtes und möglicherweise für einen Patienten gefährliches selbsttätiges Umklappen gesichert ist. Außerdem wird so erreicht, daß selbst bei Ausübung relativ großer Kräfte die Tragsäule nicht gegenüber dem Kupplungselement verschwenkbar ist, so daß eine hohe Sicherheit und Stabilität des Infusionshalters bei in Benutzungsstellung befindlicher Tragsäule erreicht ist.

Weiter ist vorgesehen, daß das/jedes Halteelement am oberen Ende der Tragsäule durch einen nach außen vorragenden Haken gebildet ist. An diesem oder diesen Haken lassen sich ein oder mehrere Infusionsflaschen schnell und einfach einhängen und werden dann sicher gehaltert, z.B. auch innerhalb von in Fahrt befindlichen Krankentransportfahrzeugen.

Hinsichtlich der am Schwanenhals vorgesehenen weiteren Haltemittel ist bevorzugt vorgesehen, daß am freien Ende des Schwanenhalses als Halteelement(e) mindestens eine Schlauchklemme und/oder mindestens ein Haken angeordnet ist/sind. Die Schlauchklemme dient vorzugsweise zur Halterung und Führung eines Schlauchs, z.B. eines Beatmungsschlauchs oder eines Infusionsschlauchs. Der gegebenenfalls vorgesehene Haken kann zur Aufhängung einer Infusionsflasche oder zur Führung eines Infusionsschlauchs oder zur Halterung anderer Teile von medizinischen Behandlungseinrichtungen genutzt werden. Bevorzugt sind am freien Ende des Schwanenhalses beide Arten von Halteelementen vorgesehen.

Um einen in der Schlauchklemme gehaltenen und geführten Schlauch relativ zu dem Patienten möglichst günstig und in der optimalen Ausrichtung führen zu können, sieht eine weitere Ausführung der Erfindung vor, daß die Schlauchklemme am Schwanenhalsende verschwenkbar und/oder verdrehbar gelagert ist.

Um den Infusionshalter möglichst freizügig einsetzen und mit unterschiedlichen Einrichtungen, die einen Patienten tragen, verbinden zu können, ist bevorzugt das Kupplungselement zwingenartig mit einer Klemmbacke und einer Klemmschraube oder einem Klemmhebel ausgeführt. Zusätzlich besteht selbstverständlich die Möglichkeit, unterschiedliche Kupplungselemente mit im Übrigen identischen Infusionshaltern zu verbinden, um eine Anpassung des Kupplungselements an unterschiedliche, einen Patienten tragende Einrichtungen zu ermöglichen.

Eine weitere Maßnahme zur Vermeidung von losen Gegenständen auf einer den Patienten tragenden Einrichtung oder auf dem Patienten selbst wird noch vorgeschlagen, daß in der unteren Hälfte der Tragsäule auf deren Außenumfang ein mehrere Zentimeter hoher Kunststoffmantel zur Befestigung von Perfusoren angebracht ist.

Schließlich ist erfindungsgemäß noch bevorzugt vorgesehen, daß zumindest die Tragsäule, das Kupplungselement und der Schwanenhals aus Edelstahl oder Aluminium bestehen. Diese Materialien sind korrosionsfrei und stabil, so daß sie die gestellten Anforderungen für die einzelnen Bauteile innerhalb des Infusionshalters erfüllen. Die weiteren Einzelteile des Infusionshalters können je nach Belastung ebenfalls Metallteile oder alternativ auch kostengünstigere Kunststoffteile sein. Die Kunststoffteile bestehen dabei vorzugsweise aus einem solchen Kunststoff, der für eine bedarfsweise Sterilisation des Infusionshalters ausreichend chemikalien- und/oder hitzebeständig ist.

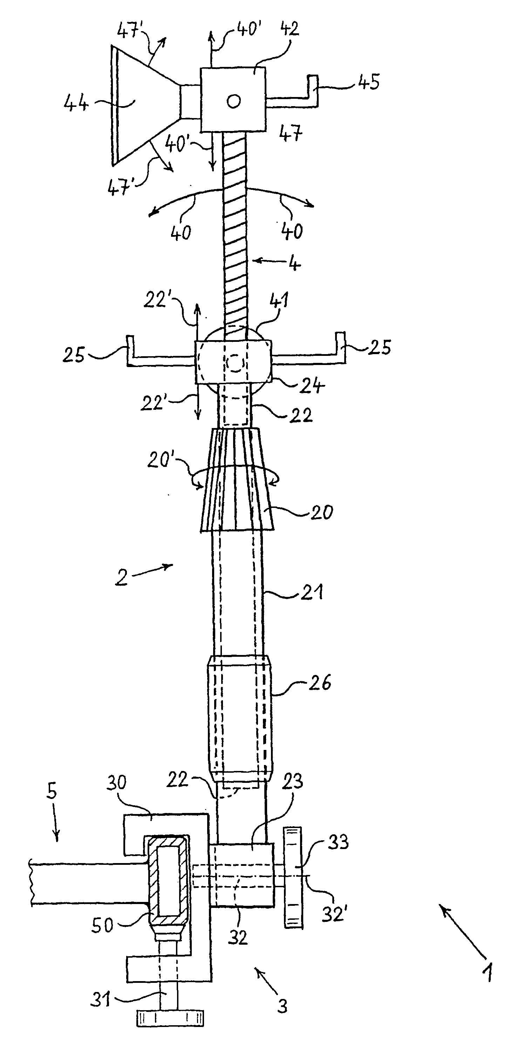

Ein Ausführungsbeispiel der Erfindung wird im folgenden anhand einer Zeichnung erläutert. Die einzige Figur der Zeichnung zeigt einen Infusionshalter in Seitenansicht in einer schematisierten Darstellung.

Wie die Zeichnungsfigur zeigt, besteht das dargestellte Ausführungsbeispiel des Infusionshalters 1 im wesentlichen aus einer Tragsäule 2, einem Kupplungselement 3 und einem Schwanenhals 4.

Die Tragsäule 2 ist hier als Teleskoprohr mit zwei ineinander geführten Rohrabschnitten ausgeführt, von denen der äußere Rohrabschnitt mit dem größeren Durchmesser den unteren Säulenteil 21 und der innere, den kleineren Durchmesser aufweisende Rohrabschnitt den oberen Säulenteil 22 bildet. Die beiden Säulenteile 21, 22 sind ineinander geführt und gegeneinander verschiebbar, wie durch die Bewegungspfeile 22' am oberen Ende des oberen Säulenteils 22 angedeutet ist. Zur Fixierung einer gewünschten Verschiebungsstellung des oberen Säulenteils 22 relativ zum unteren Säulenteil 21 dient ein Feststeller 20, hier eine Überwurfmutter, die im Sinne des Drehpfeils 20' zwischen einer Lösestellung und einer Arretierungsstellung verdrehbar ist.

An ihrem unteren Ende ist die Tragsäule 2 mit dem Kupplungselement 3 verbunden. Das Kupplungselement 3 ist zwingenartig mit einer in der Ansicht etwa C-förmigen Klemmbacke 30 und einer darin in einer Gewindebohrung verdrehbaren Klemmschraube 31 ausgeführt. Mittels dieses Kupplungselements 3 kann der Infusionshalter 1, wie in der Zeichnung dargestellt, mit einem tragfähigen Bauteil, beispielsweise einem Längsholm 50, einer einen Patienten tragenden Einrichtung 5, wie Krankentrage oder Krankenbett, lösbar und zugleich ausreichend fest und sicher verbunden werden.

Die Verbindung zwischen dem unteren Ende der Tragsäule 2 und dem Kupplungselement 3 ist als Schwenkgelenk 32 ausgestaltet, dessen Schwenkachse 32' in einer im montierten Zustand des Kupplungselements 3 etwa horizontalen Richtung in der Zeichnungsebene verläuft. Das Schwenkgelenk 32 ist mittels einer Festsstellschraube 33 in gewünschten Stellungen festlegbar. Bei gelöster Feststellschraube 33 ist die Tragsäule 2 relativ zum Kupplungselement 3 um die Schwenkachse 32' um wenigstens 90° zwischen der in der Zeichnung gezeigten, etwa vertikal nach oben verlaufenden Benutzungsstellung und einer etwa horizontalen,. senkrecht zur Zeichnungsebene verlaufenden Ruhe- oder Nichtbenutzungsstellung verschwenkbar.

Durch vollständiges Lösen der Feststellschraube 33 kann die Tragsäule von dem Kupplungselement 3 vollständig getrennt werden und an anderer Stelle eingesetzt werden; außerdem besteht so die Möglichkeit, die Tragsäule 2 je nach Bedarf mit unterschiedlichen, insbesondere unterschiedlich großen oder unterschiedlich geformten Kupplungselementen 3 zu verbinden.

Am oberen Ende der Tragsäule 2, hier am oberen Ende des oberen Säulenteils 22, ist ein Kopfstück 24 angebracht, das in etwa eine hohlzylindrische Form mit einem gegenüber dem Außendurchmesser des oberen Säulenteils 22 vergrößerten Außendurchmesser hat. In zwei entgegengesetzte Richtungen erstrecken sich von dem Kopfstück 24 zwei erste Halteelemente 25 in Form von Haken etwa radial nach außen. An diesen Haken können beispielsweise Infusionsflaschen und/oder -schläuche aufgehängt werden.

Ausgehend vom oberen Ende der Tragsäule 2 erstreckt sich der Schwanenhals 4 nach oben. Der Schwanenhals 4 ist in sich gelenkig oder biegsam und dadurch in seinem Verlauf verstellbar, beispielsweise zu den Seiten hin in einer oder mehreren Krümmungen verbiegbar, wie durch die Bewegungspfeile 40 angedeutet ist. Außerdem ist der Schwanenhals 4 relativ zu der Tragsäule 2, hier relativ zu dem oberen Säulenteil 22, verschiebbar. Dazu ist der Schwanenhals 4 in seiner in der Zeichnung gezeigten gestreckten, also im wesentlichen geradlinigen Stellung in das hohle Innere der Tragsäule 2 einschiebbar und aus diesem herausziehbar. Zur Fixierung des Schwanenhalses 4 in gewünschten Ausziehstellungen relativ zum das obere Ende der Tragsäule 2 bildenden Kopfstück 24 ist in diesem in einer Gewindebohrung eine weitere Feststellschraube 41 geführt. In eingeschraubter Stellung fixiert das innere Ende der Feststellschraube 41 den Schwanenhals 4; in ihrer losgedrehten Stellung gibt die Feststellschraube 41 den Schwanenhals 4 frei, so daß er relativ zum Kopfstück 24 nach oben oder unten verschoben werden kann.

Am freien, oberen Ende des Schwanenhalses 4 ist ein Endstück 42 angebracht, das hier ebenfalls eine zylindrische Form hat. Zur einen Seite, in der Zeichnung nach rechts, des Endstücks 42 erstreckt sich als weiteres Halteelement ein Haken 45, der ebenfalls zum Aufhängen einer Infusionsflasche oder zum Führen eines Infusionsschlauchs oder eines anderen Teils einer medizinischen Behandlungseinrichtung genutzt werden kann.

Zu der anderen Seite des Endstücks 42 hin erstreckt sich ein weiteres Halteelement in Form einer Schlauchklemme 44. Diese Schlauchklemme 44 dient beispielsweise zur Halterung und Führung eines Beatmungsschlauchs, der von einem Beatmungsgerät zu einem Patienten führt. Damit der von der Schlauchklemme 44 gehaltene Schlauch eine relativ zum Patienten günstige Lage und Führung erhält, wird der Schwanenhals 4 in seiner Höhenstellung sowie in seiner Biegung bedarfsgerecht eingestellt, wobei er diese Stellung nach Festdrehen der Feststellschraube 41 beibehält. Zur weiteren Verbesserung der Anpassungsmöglichkeiten der Position der Schlauchklemme 44 ist bei dem dargestellten Ausführungsbeispiel des Infusionshalters 1 vorgesehen, daß die Schlauchklemme 44 mittels eines Schwenkgelenks 47 in dem Endstück 42 gehaltert ist. Hierdurch wird eine Verschwenkung der Schlauchklemme 44 im Sinne der daran eingezeichneten Schwenkpfeile 47' um die senkrecht zur Schwanenhals-Längsachse verlaufende Schwenkachse des Schenkgelenks 47 ermöglicht. Zusätzlich kann die Schlauchklemme 44 zusammen mit dem Endstück 42 und dem gesamten übrigen Schwanenhals 4 um dessen Mittelachse relativ zum Kopfstück 24 verdreht werden, solange die Feststellschraube 41 gelockert ist. Damit werden alle denkbaren Stellung und Ausrichtungen der Schlauchklemme 44, die in der Praxis benötigt werden, ermöglicht.

Im unteren Teil der Tragsäule 2 ist auf dem Außenumfang des unteren Säulenteils 21 schließlich noch ein in der Praxis mehrere Zentimeter hoher Kunststoffmantel 26 aufgebracht, der zur Befestigung von Perfusoren genutzt werden kann.

Wie die Zeichnung veranschaulicht, kann der Infusionshalter 1 in maximal ausgezogener Stellung des oberen Säulenteils 22 und des Schwanenhalses 4 eine für alle Anwendungszwecke ausreichende maximale Länge erhalten, in der Praxis in der Größenordnung von etwa 1 bis 1,5 m. Wenn der Infusionshalter eine kleinere Länge erhalten soll, kann eine entsprechende Verkürzung durch Einschieben des oberen Säulenteils 22 in den unteren Säulenteil 21 und des Schwanenhalses 4 in den oberen Säulenteil 22 bewirkt werden. Bei Nichtbenutzung des Infusionshalters 1 kann dessen Länge durch vollständiges Zusammenschieben von unterem und oberen Säulenteil 21, 22 und des Schwanenhalses 4 auf eine minimale Länge gebracht werden, die nur noch etwas mehr als ein Drittel der maximal möglichen Länge des Infusionshalters 1 beträgt. In dieser Stellung mit minimaler Länge läßt sich der Infusionshalter 1 platzsparend unterbringen. Beispielsweise kann die Tragsäule 2 mit dem Schwanenhals 4 des Infusionshalters 1 in dessen an einer Tage angebrachtem Zustand nach Verschwenken um die Schwenkachse 32' um etwa 90° in eine Lage parallel zu dem Längsholm 50 gebracht werden, wo der Infusionshalter 1 ohne zu stören an der den Patienten tragenden Einrichtung 5 verbleiben kann.

Der Infusionshalter 1 ist schnell und einfach mit seinem Kupplungselement 3 mit einer einen Patienten tragenden Einrichtung 5 verbindbar und ebenso leicht und schnell von dieser bei Bedarf trennbar. Zudem ist der Infusionsständer 1 vielfältig verstellbar, was eine Anpassung an alle Bedarfsfälle ermöglicht, die in der Praxis auftreten. Dabei bietet er eine Vielzahl von flexiblen Halterungsmöglichkeiten, was das lose Herumliegen von Teilen von medizinischen Behandlungseinrichtungen verhindert. Wenn der Infusionshalter 1 in seinen tragenden Teilen aus Leichtmetall, insbesondere Aluminium, und in seinen sonstigen Teilen aus einem ausreichend stabilen Kunststoff besteht, trägt er zum Gesamtgewicht der den Patienten tragenden Einrichtung 5 nicht merklich bei, so daß auch keine zusätzliche Gewichtsbelastung für die Personen auftritt, die die den Patienten tragende Einrichtung 5, wie Krankentrage, tragen und bewegen müssen.

## Patentansprüche

1. Infusionshalter (1) zur Anbringung an einen Patienten tragenden Einrichtungen (5), wie Krankentragen oder
- betten, mit einer aus einem oberen und unteren Säulenteil (21, 22) bestehenden Tragsäule (2), die an ihrem unteren Ende ein Kupplungselement (3) zur Verbindung mit der den Patienten tragenden Einrichtung (5) und an ihrem oberen Ende mindestens ein Halteelement (25) für eine Infusionsflasche aufweist,
**dadurch gekennzeichnet,**
- **daß** der Infusionshalter (1) zusätzlich mit einem vom oberen Ende der Tragsäule (2) ausgehenden, in seinem Verlauf verstellbaren, gelenkigen oder biegsamen Schwanenhals (4) ausgestattet ist,
- **daß** der Schwanenhals (4) an seinem freien Ende ein oder mehrere weitere Halteelemente (44, 45), insbesondere für einen Beatmungsschlauch und/oder für eine Infusionsflasche und/oder für andere Teile einer medizinischen Behandlungseinrichtung, aufweist,
- **daß** die Tragsäule (2) rohrförmig mit einem hohlen Inneren ausgeführt und oben offen ist und
- **daß** der Schwanenhals (4) in gestreckter Stellung in das Innere der Tragsäule (2) einschiebbar und aus dieser herausziehbar ist.

2. Infusionshalter nach Anspruch 1, **dadurch gekennzeichnet, daß** der Schwanenhals (4) in unterschiedlichen Ausziehstellungen relativ zur Tragsäule (2) durch ein Feststellmittel (41) fixierbar ist.

3. Infusionshalter nach einem der vorhergehenden Ansprüche, **dadurch gekennzeichnet, daß** die Tragsäule (2) als Teleskoprohr mit mindestens zwei ineinander geführten und gegeneinander verschiebbaren sowie fixierbaren Rohrabschnitten (21, 22) ausgebildet ist.

4. Infusionshalter nach Anspruch 3, **dadurch gekennzeichnet, daß** das Kupplungselement (3) mit einem äußeren, den unteren Tragsäulenteil bildenden Rohrabschnitt (21) verbunden ist und daß das an der Tragsäule (2) vorgesehene Halteelement (25) an einem inneren, den oberen Tragsäulenteil bildenden Rohrabschnitt (22) angebracht ist.

5. Infusionshalter nach einem der vorhergehenden Ansprüche, **dadurch gekennzeichnet, daß** die Tragsäule (2) und das Kupplungselement (3) mittels eines feststellbaren Schwenkgelenks (32) miteinander verbunden sind, wobei die Schwenkachse (32') des Gelenks (32) senkrecht zur Längsrichtung der Tragsäule (2) verläuft und wobei in dem an einer einen Patienten tragenden Einrichtung (5) angebrachten Zustand des Infusionshalters (1) die Tragsäule (2) um die Gelenk-Schwenkachse (32') um mindestens 90° zwischen einer etwa vertikal nach oben verlaufenden Benutzungsstellung und einer etwa horizontalen, parallel zu der den Patienten tragenden Einrichtung (5) verlaufenden Nichtbenutzungsstellung verschwenkbar ist.

6. Infusionshalter nach Anspruch 5, **dadurch gekennzeichnet, daß** zumindest in der Benutzungsstellung der Tragsäule (2) bei festgestelltem Gelenk (32) zwei Teile des Gelenks (32) in einem formschlüssigen Eingriff miteinander stehen.

7. Infusionshalter nach einem der vorhergehenden Ansprüche, **dadurch gekennzeichnet, daß** das/jedes Halteelement (25) am oberen Ende der Tragsäule (2) durch einen nach außen vorragenden Haken gebildet ist.

8. Infusionshalter nach einem der vorhergehenden Ansprüche, **dadurch gekennzeichnet, daß** am freien Ende des Schwanenhalses (4) als Halteelement(e) (44, 45) mindestens eine Schlauchklemme (44) und/oder mindestens ein Haken (45) angeordnet ist.

9. Infusionshalter nach Anspruch 8, **dadurch gekennzeichnet, daß** die Schlauchklemme (44) am Schwanenhals-Ende verschwenkbar und/oder verdrehbar gelagert ist.

10. Infusionshalter nach einem der vorhergehenden Ansprüche, **dadurch gekennzeichnet, daß** das Kupplungselement (3) zwingenartig mit einer Klemmbacke (30) und einer Klemmschraube (31) oder einem Klemmhebel ausgeführt ist.

11. Infusionshalter nach einem der vorhergehenden Ansprüche, **dadurch gekennzeichnet, daß** in der unteren Hälfte der Tragsäule (2) auf deren Außenumfang ein mehrere Zentimeter hoher Kunststoffmantel (26) zur Befestigung von Perfusoren angebracht ist.

12. Infusionshalter nach einem der vorhergehenden Ansprüche, **dadurch gekennzeichnet, daß** zumindest die Tragsäule (2), das Kupplungselement (3) und der Schwanenhals (4) aus Edelstahl oder Aluminium bestehen.

## Claims

1. Infusion holder (1) for being mounted to devices (5) that support a patient, such as stretchers or hospital beds, comprising a supporting column (2) consisting of an upper and a lower column section (21, 22), said supporting column (2) comprising, at its lower end, a coupling element (3) for being joined to the device (5) that supports the patient and, at its upper end, at least one holding element (25) for holding an infusion bag,
**characterized in that**,
- the infusion holder (1) is additionally equipped with a gooseneck (4), which starts at the upper end of the supporting column (2) and is adjustable, articulated or flexible in its course,
- the gooseneck (4), at its free end, comprises one or more additional holding elements (44, 45), particularly for a breathing tube and/or an infusion bag and/or for other parts of a medical treatment device,
- the supporting column (2) is designed in the shape of a tube with a hollow interior region that is open at the top, and
- the gooseneck (4), in its straight position, can be retracted inside and pulled out from the interior region of the supporting column (2).

2. Infusion holder according to Claim 1, **characterized in that** the gooseneck (4) can be fixed in various positions of extraction in relation to the supporting column (2) by using a locking device (41).

3. Infusion holder according to anyone of the preceding claims, **characterized in that** the supporting column (2) is a telescopic tube with at least two tube sections (21, 22) that are passed one within the other and can be pushed and fixed in position in relation to each other.

4. Infusion holder according to Claim 3, **characterized in that** the coupling element (3) is connected to an outer tube section (21) that forms the lower part of the supporting column, and that the holding element (25) provided on the supporting column (2) is attached to an inner tube section (22) that forms the upper part of the supporting column.

5. Infusion holder according to anyone of the preceding claims, **characterized in that** the supporting column (2) and the coupling element (3) are connected to each other by means of a lockable swivel joint (32), with the swivel axis (32') of the joint (32) extending perpendicular to the longitudinal direction of the supporting column (2) and with the supporting column (2), while the infusion holder (1) is mounted to the device (5) that supports the patient, being capable of being swivelled about the swivel axis (32') of the joint by at least 90 degrees from a position of use that is extending in an approximately vertical upward direction to a position of non-use that is extending in an approximately horizontal direction parallel to the device (5) that supports the patient, and vice versa.

6. Infusion holder according to Claim 5, **characterized in that**, at least in the position of use of the supporting column (2) and with the joint (32) being locked, two parts of the joint (32) are in positive engagement with one another.

7. Infusion holder according to anyone of the preceding claims, **characterized in that** the/each holding element (25) at the upper end of the supporting column (2) is formed by a hook that is projecting in an outward direction.

8. Infusion holder according to anyone of the preceding claims, **characterized in that** at least one line clamp (44) and/or at least one hook (45) is/are arranged as holding element(s) (44, 45) at the free end of the gooseneck (4).

9. Infusion holder according to Claim 8, **characterized in that** the line clamp (44) at the end of the gooseneck is supported such that it can be swivelled and/or turned.

10. Infusion holder according to anyone of the preceding claims, **characterized in that** the coupling element (3) is designed in the way of a clamp with a clamping jaw (30) and a clamping screw (31) or a clamping lever.

11. Infusion holder according to anyone of the preceding claims, **characterized in that** a plastic jacket (26), several centimetres in height, is arranged in the lower half and on the outer circumference of the supporting column (2), wherein said plastic jacket (26) can be used for the attachment of perfusors.

12. Infusion holder according to anyone of the preceding claims, **characterized in that** at least the supporting column (2), the coupling element (3) and the gooseneck (4) are made of stainless steel or aluminium.

## Revendications

1. Support de perfusion (1) à monter sur des dispositifs (5) soutenant un patient, tels que des civières ou des lits pour malades, comportant une colonne de support (2) composée de parties de colonne supérieure et inférieure (21, 22), laquelle comporte, en sa partie inférieure, un élément de couplage (3) pour raccordement au dispositif (5) soutenant le patient et, en sa partie supérieure, au moins un élément de maintien (25) pour une bouteille de perfusion,
**caractérisé en ce que**
- le support de perfusion (1) est additionnellement équipé d'un col de cygne (4) qui part de la partie supérieure de la colonne de support (2) et est ajustable, articulé ou pliable sur son cours;
- le col de cygne (4) comporte, en son extrémité libre, un ou plusieurs autres éléments de maintien (44, 45), en particulier pour un tuyau de respiration et/ou pour une bouteille de perfusion et/ou pour d'autres parties d'un dispositif de traitement médical;
- la colonne de support (2) est réalisée sous une forme tubulaire avec un intérieur creux et est ouverte en haut et
- le col de cygne (4), en position étirée, peut être glissé à l'intérieur de la colonne de support (2) et peut en être retiré.

2. Support de perfusion selon la revendication 1, **caractérisé en ce que** le col de cygne (4) peut être fixé par un moyen de blocage (41) dans différentes positions d'allongement relativement à la colonne de support (2).

3. Support de perfusion selon l'une des revendications précédentes, **caractérisé en ce que** la colonne de support (2) est réalisée comme tube téléscopique comprenant au moins deux segments de tube (21, 22) fixables, coulissants l'un dans l'autre et déplaçables l'un contre l'autre.

4. Support de perfusion selon la revendication 3, **caractérisé en ce que** l'élément de couplage (3) est relié à un segment de tube (21) extérieur qui constitue la partie inférieure de la colonne de support et **en ce que** l'élément de maintien (25) prévu sur la colonne de support (2) est monté sur un segment de tube (22) intérieur qui constitue la partie supérieure de la colonne de support.

5. Support de perfusion selon l'une des revendications précédentes, **caractérisé en ce que** la colonne de support (2) et l'élément de couplage (3) sont reliés entre eux au moyen d'une articulation pivotante (32) blocable, l'axe de pivotement (32') de l'articulation (32) étant perpendiculaire à la direction longitudinale de la colonne de support (2) et la colonne de support (2), dans l'état monté sur un dispositif (5) soutenant un patient, peut pivoter, autour de l'axe de pivotement (32') de l'articulation, d'au moins 90° entre une position d'utilisation à peu près verticale allant vers le haut et une position de non-utilisation à peu près horizontale, parallèle au dispositif (5) soutenant le patient.

6. Support de perfusion selon la revendication 5, **caractérisé en ce que**, du moins dans la position d'utilisation de la colonne de support (2), l'articulation (32) étant bloquée, deux parties de l'articulation (32) sont engagées l'une dans l'autre par complémentarité de forme.

7. Support de perfusion selon l'une des revendications précédentes, **caractérisé en ce que** le/chaque élément de maintien (25) est constitué, dans la partie supérieure de la colonne de support (2), par un crochet faisant saillie vers l'extérieur.

8. Support de perfusion selon l'une des revendications précédentes, **caractérisé en ce qu'**au moins une pince pour tuyaux souples (44) et/ou au moins un crochet (45) sont situés à l'extrémité libre du col de cygne (4) en tant qu'élément(s) de maintien (44, 45).

9. Support de perfusion selon la revendication 8, **caractérisé en ce que** la pince pour tuyaux souples (44) est montée de manière à pouvoir pivoter et/ou tourner à l'extrémité du col de cygne.

10. Support de perfusion selon l'une des revendications précédentes, **caractérisé en ce que** l'élément de couplage (3) est réalisé comme un crampon avec une mâchoire de serrage (30) et une vis de serrage (31) ou un levier de serrage.

11. Support de perfusion selon l'une des revendications précédentes, **caractérisé en ce qu'**une enveloppe de matière plastique (26) haute de plusieurs centimètres est montée dans la moitié inférieure de la colonne de support (2), sur son pourtour extérieur, pour fixer des perfuseurs.

12. Support de perfusion selon l'une des revendications précédentes, **caractérisé en ce que** du moins la colonne de support (2), l'élément de couplage (3) et le col de cygne (4) sont composés d'acier fin ou d'aluminium.
